# EUROPEAN PATENT APPLICATION

(11) **EP 1 609 778 A1**
(43) Date of publication of application: **28.12.2005**
(21) Application number: 04722662.6
(22) Date of filing: 23.03.2004
(51) Int. Cl.: C07C 255/23, C07C 253/30, C07C 253/34

(54) **PROCESS FOR PRODUCTION OF 2-CYANO-3-HYDROXY-N-(4-TRI- FLUOROMETHYLPHENYL)HEPT-2-EN-6-YNAMIDE AND PROCESS FOR PRODUCTION OF POLYMORPHS THEREOF**

(30) Priority: 24.03.2003 JP 2003081335; 20.06.2003 JP 2003176706
(71) Applicant: Astellas Pharma Inc., Tokyo 103-8411 (JP)
(72) Inventor: OMORI, Hiroki, Amagasaki-shi, Hyogo 660-0881 (JP); KUBOTA, Ariyoshi, c/o Astellas Pharma Inc., Chuo-Ku, Tokyo 103-8411 (JP); KAWAKAMI, Takeshi, c/o Astellas Pharma Inc., Chuo-ku, Tokyo 103-8411 (JP); FUJII, Yosuke, c/o Astellas Pharma Inc., Chuo-ku, Tokyo 103-8411 (JP); MATSUMOTO, Ikuo, c/o Astellas Pharma Inc., Chuo-ku, Tokyo 103-8411 (JP); KITAYAMA, Masato, c/o Astellas Pharma Inc., Chuo-ku, Tokyo 103-8411 (JP); GOTO, Shunsuke, c/o Astellas Pharma Inc., Chuo-ku, Tokyo 103-8411 (JP); HIRABAYASHI, Satoshi, Higashinada-ku, Kobe-shi, Hyogo 658-0001 (JP)
(74) Representative: Gille Hrabal Struck Neidlein Prop Roos
(86) International application number: PCT/JP2004/003904
(87) International publication number: WO 2004/085383

(57) **Abstract**

A compound (II) is reacted with a compound (III) or a reactive derivative at the carboxyl group thereof to obtain a compound (IV), which is reacted with a mixed acid anhydride of a compound (V) to prepare a compound (I). In recrystallization of the compound (I), temperature for the recrystallization and/or time for the crystal precipitation is controlled to selectively produce A-form crystals, B-form crystals and C-form crystals of the compound (I).
[Chemical formula 1]

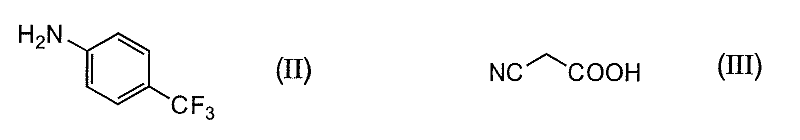

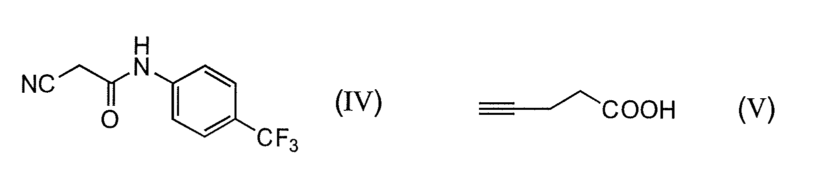

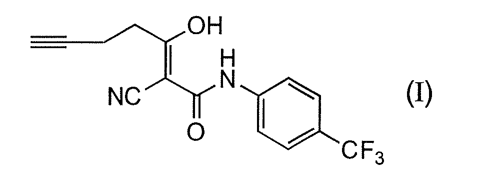

## Description

### Technical Field

The present invention relates to a novel method for production of 2-cyano-3-hydroxy-N-(4-trifluoromethylphenyl)hept-2-en-6-ynamide having immunosuppressive activity (hereinafter referred to as "compound (I)") represented by the formula (I): 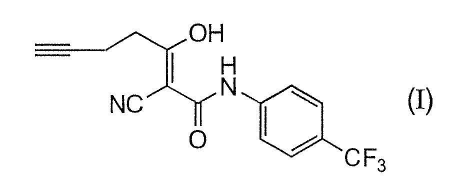 and a method for production of polymorphs thereof.

More specifically, the present invention relates to a novel method for preparing the compound (I) having immunosuppressive activity, and a method for selectively obtaining anyone of polymorphs comprising A-form crystals, B-form crystals and C-form crystals of the compound (I).

### Background Art

The compound (I) is useful for treatment of rheumatoid arthritis, immune or nonimmune chroic inflammatory diseases such as graft versus host disease, reactions by transplantation and uveitis, as well as cancer. A preparation method thereof has been described in Japanese Unexamined Patent Publication No. HEI 5-310672 (Patent Document 1).

The compound (I) is specifically described in Preparation Example 14 of the above Patent Document 1, in which polymorphs of the compound and a method for selectively obtaining anyone of the same have not been reported.

### Disclosure of the Invention

In the preparation method of the compound (I) described in Patent Document 1, n-butyl lithium, which is highly flammable, is used in an amount as large as 3 equivalent weight with respect to a material compound and a reaction temperature needs to be controlled at -78°C. It has been a problem since the method is not suitable for manufacture on an industrial scale.

Further, for preparing a safe and effective drug, homogeneity in physical property of the drug is essential. Even the same compounds may have different physical properties if their crystal forms are different. If the crystal form is not controlled, i.e., a single crystal form cannot be formed constantly, the resulting drug may irregularly vary in quality batch by batch. Therefore, manufacture of a substance having uniform physical properties, that is, control of the crystal form, is an extremely important subject for quality control of the drug.

The present invention has been achieved to solve the above-described problem and subject and based on the finding that there exist polymorphs of the compound (I). Accordingly, the present invention provides a method for preparing the compound (I) suitable for manufacture on an industrial scale and a method for selectively obtaining anyone of the polymorphs of the compound (I).

With the intention of establishing a method for preparing the compound (I) free from the above-described problem, the inventors of the present invention have carried out eager study and found that the above-described problem is solved by reacting a compound (IV) represented by the formula (IV): 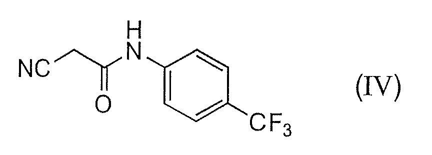 with a mixed acid anhydride of a compound (V) represented by the formula (V): 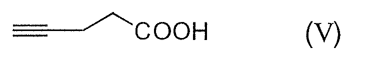 thereby completing the present invention.

Further, in the course of study on a purification method of the compound (I), the inventors of the present invention have also found that A-form crystals, B-form crystals and C-form crystals of the compound (I) are selectively obtained by controlling the temperature for recrystallizing the compound (I), thereby completing the present invention.

According to the present invention, provided is a method for preparing a compound (I) represented by the formula: 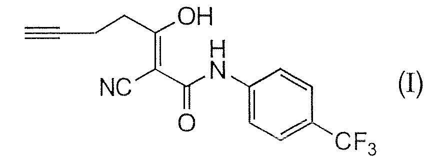 by reacting a compound (II) represented by the formula: 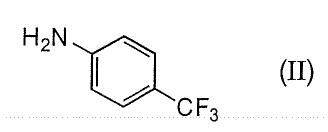 with a carboxylic acid (III) represented by the formula: 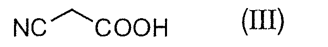 or a reactive derivative at the carboxyl group thereof to give a compound (IV) represented by the formula: 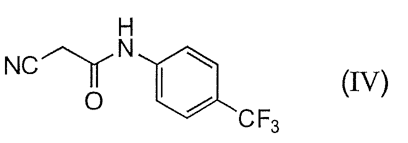 and reacting the resultant compound with a mixed acid anhydride of a compound (V) represented by the formula: 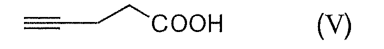 to give the compound (I).

Further, according to the present invention, provided is a method for selectively obtaining A-form crystals, B-form crystals or C-form crystals of the compound (I) by controlling the temperature for recrystallizing the compound (I) during the recrystallization of the compound (I).

### Brief Explanation of Drawings

Fig. 1 is an IR spectrum of A-form crystals of the compound (I);
Fig. 2 is an IR spectrum of B-form crystals of the compound (I);
Fig. 3 is an IR spectrum of C-form crystals of the compound (I);
Fig. 4 shows measurement results of a DSC endothermic test of the A-form crystals of the compound (I);
Fig. 5 shows measurement results of a DSC endothermic test of the B-form crystals of the compound (I);
Fig. 6 shows measurement results of a DSC endothermic test of the C-form crystals of the compound (I);
Fig. 7 is an X-ray diffraction pattern of the A-form crystals of the compound (I);
Fig. 8 is an X-ray diffraction pattern of the B-form crystals of the compound (I); and
Fig. 9 is an X-ray diffraction pattern of the C-form crystals of the compound (I).

### Best Mode for Carrying Out the Invention

The above-described preparation method is represented by the following equation.

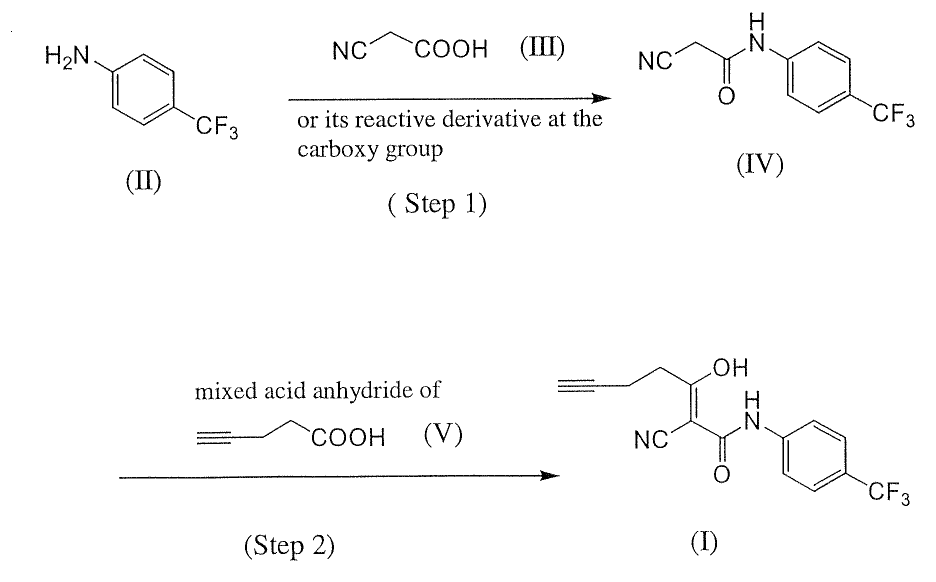

The above preparation method is explained below.

### First step:

A reaction in the first step is conducted by reacting a compound (II) with a compound (III) or a reactive derivative at the carboxyl group thereof.

The reactive derivative at the carboxyl group of the compound (III) may be the acid halide, acid azide, acid anhydride, mixed acid anhydride, active amide, active ester and the like.

Concrete examples of the reactive derivative include: acid halide; acid azide; symmetric acid anhydride; asymmetric acid anhydride with acid such as substituted phosphoric acid (for example, dialkyl phosphite, phenyl phosphate, diphenyl phosphate, dibenzyl phosphate or halogenated phosphate), dialkyl phosphate, sulfurous acid, thiosulfuric acid, sulfuric acid, sulfonic acid (for example, methansulfonic acid), aliphatic carboxylic acid (for example, acetate, propionic acid, butyric acid, isobutyric acid, pivalic acid, pentanoic acid, isopentanoic acid, 2-ethylbutyric acid or trichloroacetic acid) or aromatic carboxylic acid (for example, benzoic acid); mixed acid anhydride prepared from chloro(lower)alkyl carbonate or cycloalkyl chlorocarbonate; active amide with imidazole, 4-substituted imidazole, dimethylpyrazole, triazole or tetrazole; active ester (for example, cyanomethyl ester, methoxymethyl ester, dimethyliminomethyl ((CH₃)₂N⁺=CH⁻) ester, vinyl ester, propargyl ester, p-nitrophenyl ester, 2,4-dinitrophenyl ester, trichloropllenyl ester, pentachlorophenyl ester, mesylphenyl ester, phenylazophenyl ester, phenyl thioester, p-nitrophenyl thioester, p-cresyl thioester, carboxymethyl thioester, pyranyl ester, pyridyl ester, piperidyl ester or 8-quinolyl thioester); or ester with N-hydroxy compounds (for example, N,N-dimethylhydroxylamine, 1-hydroxy-2-(1H)-pyridone, N-hydroxysuccinimide, N-hydroxyphthalimide or 1-hydroxy-1H-benzotriazole).

Each of the above-described reactive derivatives may be obtained by a usual method.

The reaction of the first step is usually carried out in a solvent with cooling or heating.

Examples of the solvent used in this reaction include esters such as ethyl acetate, ethers such as tetrahydrofuran, diethyl ether and dioxane, aprotic polar solvents such as acetone, acetonitrile, N,N-dimethylformamide and pyridine, halogenated hydrocarbons such as 1,2-dichloroethane, chloroform and chlorobenzene, saturated or unsaturated hydrocarbons such as hexane, benzene and toluene or a mixture of these solvents.

If the compound (III) is used in the form of free acid in the reaction of the first step, it is preferable to carry out the reaction in the presence of a normal condensing agent, for example, N,N'-dicyclohexylcarbodiimide, N-cyclohexyl-N'-morpholinoethylcarbodiimide, N-cyclohexyl-N'-(4-diethylaminocyclohexyl)carbodiimide, N,N'-diethylcarbodiimide, N,N'-diisopropylcarbodiimide, N-ethyl-N'-(3-dimethylaminopropyl)carbodiimide, N,N'-carbonyl bis-(2-methylimidazole), pentamethyleneketene-N-cyclohexylimine, diphenylketene-N-cyclohexylimine, ethoxyacetylene, 1-alkoxy-1-chloroethylene, trialkylphosphite, ethylpolyphosphate, isopropylpolyphosphate, phosphorus oxychloride (phosphoryl chloride), phosphorus trichloride, diphenylphosphorylazide, thionyl chloride, oxalyl chloride, lower alkyl haloformate (for example, ethylchloroformate, isopropylchloroformate and the like), triphenylphosphine, 2-ethyl-7-hydroxybenzisoxazolium salt, 2-ethyl-5-(m-sulfophenyl)-isoxazolium hydroxide inner salt, benzotriazol-1-yloxy-tris(dimethylamino)phosphonium hexafluorophosphate, 1-(p-chlorobenzenesulfonyloxy)-6-chloro-1H-benzotriazole, or in the presence of a so-called Vilsmeier reagent obtained by a reaction between N,N-dimethylformamide and thionyl chloride, phosgene, trichloromethylchloroformate, phosphorus oxychloride or the like.

If the compound (III) is used in the form of free acid, the reaction may be carried out in the presence of an acid such as inorganic acid, organic acid and Lewis acid.

Examples of the inorganic acid include sulfuric acid, hydrochloric acid and the like. Examples of the organic acid include p-toluenesulfonic acid, benzenesulfonic acid, methansulfonic acid, ethansulfonic acid, sulfosalicylic acid and the like. Examples of the Lewis acid include aluminum chloride, diethyl ether boron trifluoride complex, tin tetrachloride, titanium tetrachloride, yttrium triflate, scantium triflate and the like. Particularly preferable is p-toluenesulfonic acid.

In general, the reaction in the presence of acid is preferably conducted in a nonpolar solvent which is not mixed with water, e.g., benzene, toluene, xylene, chlorobenzene or the like, with heating, while removing water generated with the progress of the reaction out of the reaction system, or in the presence of a dehydrating agent such as a molecular sieve.

Further, this reaction may be carried out in the presence of an inorganic base such as alkali metal hydrogen carbonates, alkali metal carbonates and alkali metal hydroxides or in the presence of an organic base such as tri(lower)alkylamine, pyridine, N-(lower)alkylmorpholine, N,N-di(lower)alkylaminopyridine, N,N-di(lower)alkylaniline and N,N-di(lower)alkylbenzylamine.

The compound (IV) obtained in the above-described manner may be isolated and purified by a usual method. However, it may be used in a reaction of the following second step without particular isolation or purification.

### Second step:

A reaction in the second step is carried out by reacting the compound (IV) obtained as described above with a mixed acid anhydride of a compound (V) in a solvent.

As the mixed acid anhydride of the compound (V), preferably is a mixed acid anhydride with chloro(lower)alkyl carbonate or cycloalkyl chlorocarbonate. Examples of the chloro(lower)alkyl carbonate include methyl chlorocarbonate, ethyl chlorocarbonate, n-propyl chlorocarbonate, isopropyl chlorocarbonate, n-butyl chlorocarbonate, s-butyl chlorocarbonate, t-butyl chlorocarbonate, isoamyl chlorocarbonate and the like. Examples of the cycloalkyl chlorocarbonate include cyclopentyl chlorocarbonate, cyclohexyl chlorocarbonate and the like. Further, bromides, fluorides and iodides converted from these chlorides may also be used.

Preparation of the mixed acid anhydride of the compound (V) is generally carried out in an organic solvent with cooling or heating in the presence of an inorganic base or an organic base. The inorganic base, organic base and organic solvent may be those shown in the above first step.

The thus obtained mixed acid anhydride of the compound (V) may be isolated and purified by a usual method. However, it may also be used as it is in a reaction with a compound (IV) without being isolated and purified.

In the same manner as the above first step, the reaction of the second step is generally conducted with cooling or heating in a solvent.

The thus obtained compound (I) may be isolated and purified by a usual method, but preferably, it is purified by recrystallization.

Examples of a solvent for the recrystallization include alcohols such as methanol, ethanol, n-propyl alcohol, isopropyl alcohol, n-butyl alcohol, t-butyl alcohol and the like, ketones such as acetone, methyl ethyl ketone and the like, esters such as methyl acetate, ethyl acetate, isopropyl acetate and the like, nitriles such as acetonitrile and the like, ethers such as tetrahydrofuran, dioxane and the like, as well as a mixed solvent of the above-listed solvents capable of being mixed with water and water as a poor solvent, and a mixed solvent of the above-listed solvent and a poor solvent, which may be an aliphatic hydrocarbon such as n-pentane, cyclopentane, n-hexane, cyclohexane, n-heptane or cycloheptane, an aromatic hydrocarbon such as benzene, toluene, xylene or the like, or disopropyl ether.

Further, as described below, the present invention allows to selectively obtain A-form crystals, B-form crystals and C-form crystals of the compound (I) by controlling the temperature for recrystallization and/ or time for crystal precipitation during the recrystallization of the compound (I).

In other words, the A-form crystals of the compound (I), characterized by having an IR (KBr) spectrum of Fig. 1, a DSC endothermic curve of Fig. 4 and an X-ray diffraction pattern of Fig. 7 and having characteristic peaks at about 6.7, about 13.4 and about 21.5° at 2 *θ* of the X-ray diffraction, are obtained by dissolving crystals of the compound (I) in a solvent, maintaining the solvent at about 55°C to 95°C with stirring, adding a poor solvent if necessary, and then filtering precipitated crystals out.

Further, the B-form crystals of the compound (I), characterized by having an IR (KBr) spectrum of Fig. 2, a DSC endothermic curve of Fig. 5 and an X-ray diffraction pattern of Fig. 8 and having characteristic peaks at about 6.2, about 12.5 and about 20.8° at 2 *θ* of the X-ray diffraction, are obtained by dissolving crystals of the compound (I) in a solvent, maintaining the solvent at about 20°C to about 45°C, preferably at about 30°C to about 40°C with stirring, adding a poor solvent if necessary, and then filtering precipitated crystals out.

Still further, the C-form crystals of the compound (I), characterized by having an IR (KBr) spectrum of Fig. 3, a DSC endothermic curve of Fig. 6 and an X-ray diffraction pattern of Fig. 9 and having characteristic peaks at about 6.2, about 12.4 and about 20.2° at 2 0 of the X-ray diffraction, are obtained by dissolving crystals of the compound (I) in a solvent, maintaining the solvent at about 0°C to about 15°C with stirring, adding a poor solvent if necessary, and then filtering precipitated crystals out.

In the above steps, the poor solvent is added when the crystals are hard to be precipitated or the crystallization needs to be accelerated.

However, in the method for selectively obtaining the polymorphs according to the present invention, temperature control during the crystallization is of consequence. Accordingly, the intended crystal form is selectively obtained preferably by dissolving the compound (I) in a solvent, maintaining the solvent at any one of the above-described temperatures and adding the poor solvent, rather than by usual recrystallization.

As the solvent used for preparing the above-described A-form crystals, B-form crystals and C-form crystals, the solvent for the recrystallization of the compound (I) described above may be used. However, in view of yield and the like, particularly preferable are alcohols such as methanol and isopropyl alcohol, or an acetone, a combination of these solvents and water as a poor solvent, or a combination of a solvent such as ethyl acetate and a poor solvent such as n-heptane.

The above-described A-form crystals are produced by recrystallization performed in the temperature range of about 55°C to about 95°C.

More specifically, if methanol is used as the recrystallization solvent to obtain the A-form crystals from crude crystals of the compound (I), for example, methanol may be used in an amount of 0.8 to 80-fold (w/w) of the compound (I) and water as the poor solvent may be used in an amount of 0.5 to 50-fold (v/v) of methanol.

If acetone is used as the recrystallization solvent, acetone may be used in an amount of 0.6 to 40-fold (w/w) of the compound (I) and water as the poor solvent may be used in an amount of 0.7 to 10-fold (v/v) of acetone.

If ethyl acetate is used as the recrystallization solvent, ethyl acetate may be used in an amount of 2.5 to 10-fold (w/w) of the compound (I) and n-heptane as the poor solvent may be used in an amount of 2 to 50-fold (v/v) of ethyl acetate.

The above-described B-form crystals are produced by recrystallization performed in the temperature range of about 20°C to about 45°C, preferably about 30°C to about 40°C.

More specifically, if methanol is used as the recrystallization solvent to obtain the B-form crystals from crude crystals of the compound (I), for example, methanol may be used in an amount of 40 to 160-fold (w/w) of the compound (I) and water as the poor solvent may be used in an amount of 0.2 to 10-fold (v/v) of methanol.

If acetone is used as the recrystallization solvent, acetone may be used in an amount of 3.5 to 60-fold (w/w) of the compound (I) and water as the poor solvent may be used in an amount of 0.2 to 5-fold (v/v) of acetone.

If ethyl acetate is used as the recrystallization solvent, ethyl acetate may be used in an amount of 4.5 to 18-fold (w/w) of the compound (I) and n-heptane as the poor solvent may be used in an amount of 3 to 20-fold (v/v) of ethyl acetate.

The above-described C-form crystals are produced by recrystallization performed in the temperature range of about 0°C to about 15°C.

More specifically, if methanol is used as the recrystallization solvent to obtain the C-form crystals from crude crystals of the compound (I), for example, methanol may be used in an amount of 80 to 400-fold (w/w) of the compound (I) and water as the poor solvent may be used in an amount of 0.1 to 5-fold (v/v) of methanol.

If acetone is used as the recrystallization solvent, acetone may be used in an amount of 7 to 80-fold (w/w) of the compound (I) and water as the poor solvent may be used in an amount of 0.1 to 5-fold (v/v) of acetone.

If ethyl acetate is used as the recrystallization solvent, ethyl acetate may be used in an amount of 10 to 45-fold (w/w) of the compound (I) and n-heptane as the poor solvent may be used in an amount of 0.1 to 5-fold (v/v) of ethyl acetate.

If the thus obtained A-form crystals, B-form crystals or C-form crystals of the compound (I) are suspended in a solvent and stirred with heating while controlling the heating temperature, the A-form crystals, B-form crystals and C-form crystals of the compound (I) can selectively be converted to different crystal forms, respectively.

That is, the A-form crystals of the compound (I) can be obtained by suspending the B-form crystals or C-form crystals of the compound (I) or a mixture thereof in a solvent, maintaining the suspension at about 55°C to 95°C with stirring, and then filtering the crystals out of the suspension.

Alternatively, the B-form crystals of the compound (I) can be obtained by suspending the A-form crystals of the compound (I) in a solvent, maintaining the suspension at about 20°C to 45°C with stirring, and then filtering the crystals out of the suspension.

As The solvent used for the mutual conversion between the above-described crystal forms, the solvents for the recrystallization of the compound (I) described above may be used. However, in view of yield and the like, particularly preferable are alcohols such as methanol and isopropyl alcohol, or an acetone, a combination of these solvents and water as a poor solvent, or a combination of a solvent such as ethyl acetate and a poor solvent such as n-heptane.

Time for stirring at a predetermined temperature required for the mutual conversion between the above-described crystal forms is not particularly limited. However, sufficient time is about 5 hours to about 72 hours in general.

More specifically, in order to convert the B-form crystals or the C-form crystals of the compound (I) or a mixture thereof into the A-form crystals, for example, methanol or ethyl acetate may be used. That is, if methanol is used as the solvent, methanol may be used in an amount of 8 to 80-fold (w/w) of the B-form crystals or the C-form crystals of the compound (I) or the mixture thereof and water as the poor solvent may be used in an amount of 0.5 to 50-fold (v/v) of methanol.

If ethyl acetate is used, ethyl acetate may be used in an amount of 2.5 to 10-fold (w/w) of the B-form crystals or the C-form crystals of the compound (I) or the mixture thereof and n-heptane as the poor solvent may be used in an amount of 2 to 50-fold (v/v) of methanol.

Next, in order to obtain the B-form crystals from the A-form crystals of the compound (I), for example, methanol or ethyl acetate may be used. That is, if methanol is used as the solvent, methanol may be used in an amount of 40 to 160-fold (w/w) of the A-form crystals of the compound (I) and water as the poor solvent may be used in an amount of 0.2 to 10-fold (v/v) of methanol.

If ethyl acetate is used, ethyl acetate may be used in an amount of 4.5 to 18-fold (w/w) of the A-form crystals of the compound (I) and n-heptane as the poor solvent may be used in an amount of 3 to 20-fold (v/v) of methanol.

The term "about" used in connection with temperature in the present specification indicates that the indicated temperature may vary by ±2°C.

The term "(lower)alkyl" used in the present specification means (C₁-C₅) alkyl.

As a result of analysis on stability of the crystal forms of the compound (I) based on the measurement results of a DSC endothermic test, it was ascertained that the crystal forms are stable in the order of B-form crystals > C-form crystals > A-form crystals.

The following Examples are incorporated herein to explain the present invention in further detail, to which the scope of the present invention is not limited.

### Examples

### Example 1

### Preparation of 4-trifluoromethyl cyanoacetoanilide (compound (IV))

In 700 L of tetrahydrofuran, 63.4 kg of cyanoacetic acid was dissolved with stirring in nitrogen atmosphere at room temperature. This solution was cooled to 0 to 10°C, and then N-methylmorpholine was added dropwise with stirring at the same temperature in about an hour. Then, 100.0 kg of 4-trifluoromethylaniline was added dropwise. To this reaction mixture, 91.3kg of isopropyl chlorocarbonate was added dropwise with stirring at the same temperature in about an hour. Further, the stirring was continued for 1 to 2 hours.

After the reaction was finished, 200 L of water was added to the reaction mixture, which was stirred and then allowed to stand for separation. An organic layer (upper layer) was washed with 16.7 % brine, and then 400 L of isopropyl alcohol was added thereto, which was concentrated under reduced pressure until the liquid amount became 400 L. To the condensed solution, 400 L of isopropyl alcohol was added, which was concentrated again under reduced pressure until the liquid amount became 400 L.

To the condensed solution, 100 L of isopropyl alcohol was added at 20 to 30°C with stirring, and then 500 L of water was added dropwise in about an hour. Then, the stirring was further continued for an hour at the same temperature. This mixture was cooled to 0 to 10°C with stirring, and then stirred at the same temperature for an hour. Precipitated crystals were collected by filtration and dried, to give 134.5 kg of the title compound (IV) in a yield of 95.0 %.

### Example 2

### Preparation of 2-cyano-3-hydroxy-N-(4-trifluoromethylphenyl)hept-2-en-6-ynamide (compound (I)) from compound (IV)

In 420 L of acetone, 61.9 kg of 4-pentynoic acid was dissolved with stirring in nitrogen atmosphere at room temperature. This solution was cooled to 10 to 15°C, to which 174.4 kg of potassium carbonate was added with stirring. This mixture was heated to 40 to 45°C and a solution of 120 kg of the compound (IV) obtained in Example 1 in acetone (540 L) was added thereto at the same temperature with heating, and then 64.5 kg of isopropyl chlorocarbonate was added dropwise in about an hour. After the dropping was finished, the solution was further stirred for 30 minutes at the same temperature.

To the reaction solution, 840 L of water was added at 10 to 45°C with stirring, and then a 17.5 % hydrochloric acid solution (mixture of 90 L of water and 90 L of concentrated hydrochloric acid) was added dropwise at 20 to 35°C for 30 minutes. After the precipitate was dissolved, the solution was further stirred for 30 minutes.

Then, to this solution, a 17.5 % hydrochloric acid solution (mixture of 45 L of water and 45 L of concentrated hydrochloric acid) was added dropwise with stirring at the same temperature in an hour, which was heated to 35 to 45°C and kept stirred for about an hour at the same temperature. The mixture was cooled to 20 to 30°C and maintained at same temperature for about two hours. To this mixture, a 17.5 % hydrochloric acid solution (mixture of 45 L of water and 45 L of concentrated hydrochloric acid) was further added dropwise at 20 to 35°C in an hour, which was heated to 35 to 45°C and stirred at the same temperature for about an hour, and then maintained at 20 to 30°C for about two hours. Precipitated crystals were collected by filtration to give 136.2 kg of the title compound (I) in a yield of 84.0 %.
¹H NMR (CDCl*₃, 200MHz) δ: 2.07 (t, J=2.7Hz, 1H), 2.64 (dt, J=2.7 and 7.0Hz, 2H), 2.88 (t, J=7.0Hz, 2H), 7.65 (s, 4H), 7.77 (br.s, 1H), 15.59 (br.s, 1H).
IR (ATR method) (cm⁻¹); 3311, 2217, 1627, 1626, 1589, 1554, 1415, 1321, 1263, 1243, 1160, 1113, 1072, 841, 658.

### Example 3

### Preparation of 2-cyano-3-hydroxy-N-(4-trifluoromethylphenyl)hept-2-en-6-ynamide (compound (I)) from compound (IV)

In 618.7 kg of tetrahydrofuran, 87.0 kg of the compound (IV) obtained in Example 1 and 56.1 kg of 4-pentynoic acid were dissolved with stirring in nitrogen atmosphere at 25 to 30°C. To this solution, 158.1 kg of pulverized potassium carbonate was added with stirring at the same temperature. While stirring the mixture at 35 to 50°C, a mixture of 93.5 kg of isopropyl chlorocarbonate and 77.3 kg of tetrahydrofuran was added dropwise in 2 hours. After the dropping was finished, the reaction mixture was stirred at the same temperature for another 2 hours. After the reaction was completed, 677.3 kg of toluene was added to the reaction mixture, which was cooled to 10 to 15°C.

A mixture of 207.1 kg of hydrochloric acid and 783 L of water was added dropwise to this reaction mixture with stirring, while maintaining the temperature of the reaction mixture not higher than 25°C. After the dropping was finished, the mixture was further stirred for 5 minutes, and then the stirring was stopped to leave the mixture to stand. After the mixture was separated into two layers, an upper layer was isolated, to which 435 L of 16.7 % brine was added and stirred at room temperature for 5 minutes. The stirring was stopped and the resulting mixture was allowed to stand. After the mixture was separated into two layers, an upper layer was isolated and concentrated under reduced pressure to obtain a concentrate of 435 L. To the concentrate, 376.3 kg of toluene was added, which was concentrated again under reduced pressure to obtain a concentrate of 435 L. While maintaining the solution temperature not higher than 40°C, 376.3 kg of toluene was added to this concentrate, followed by stirring at 20 to 30°C for an hour and another stirring at 0 to 10°C for an hour. Then, the resulting mixture was allowed to stand for 2 hours at the same temperature. Precipitated crystals were collected by filtration and washed sequentially with 150.5 kg of toluene, 174 L of an aqueous solution of 50 % isopropyl alcohol and 174 L of water, followed by drying at 40°C under vacuum. Thereby, 98.7 kg of crude crystals of the title compound (I) were obtained in a yield of 84.0 %.

The ¹HNMR spectrum and IR spectrum of the compound were measured, which were in perfect agreement with those of the compound obtained in Example 2.

### Example 4

### Preparation of 2-cyano-3-hydroxy-N-(4-trifluoromethylphenyl)hept-2-en-6-ynamide (compound (I))

In 420 L of acetone, 33.3kg of cyanoacetic acid was dissolved with stirring in nitrogen atmosphere at room temperature. To this solution, 39.6 kg of N-methylmorpholine was added dropwise with stirring at 0 to 10°C in about an hour, to which 60.0 kg of 4-trifluoromethylaniline was added dropwise. Further, 52.5 kg of isopropyl chlorocarbonate was added dropwise to the solution with stirring at the same temperature in an hour, which was then stirred at the same temperature for 1 to 2 hours to give a reaction product containing the compound (IV).

In nitrogen atmosphere at 25 to 30°C, 43.8 kg of 4-pentynoic acid was dissolved in 780 L of acetone with stirring. To this solution, 221.3 kg of pulverized potassium carbonate, the reaction product containing the compound (IV) obtained in the above and 68.5 kg of isopropyl chlorocarbonate were added with stirring at the same temperature. Then, the mixture was stirred for 1 to 2 hours while maintaining the temperature at 40°C to 55°C.

After the reaction was completed, 1200 L of water was added to the reaction mixture with stirring and the mixture was cooled to 20 to 35 °C. To the mixture, a mixture of 214.2 kg of hydrochloric acid and 180 L of water was added dropwise with stirring while maintaining the temperature of the reaction mixture at 20 to 30°C. After the dropping was finished, a mixture of 107.1 kg of hydrochloric acid and 90 L of water was further added dropwise with stirring while maintaining the temperature at 20 to 35°C. Then, the reaction mixture was stirred for an hour while maintaining the temperature thereof at 35 to 45°C, followed by stirring at 20 to 30°C for an hour. Precipitated crystals were collected by filtration and washed sequentially with 180 L of a 70 % aqueous acetone solution and 300 L of a 30 % aqueous acetone solution. Thereby, 126 kg of crude wet crystals of the compound (I) were obtained.

Part of the wet crystals was dried to measure the ¹HNMR and IR spectra, which were in perfect agreement with those of the compound (I) obtained in Example 2.

### Example 5

### Preparation of A-form crystals of compound (I)

### 5-1

In 50 mL of acetone, 5 g of crude crystals of the compound (I) obtained in Example 2 were dissolved. This solution was heated to 55°C with stirring, to which 50 mL of water was added dropwise at the same temperature in about an hour. After the dropping was completed, the mixture was further stirred at the same temperature for an hour. Precipitated crystals were collected by filtration and dried at 40°C under vacuum to give 4.35 g of A-form crystals of the compound (I) in a yield of 87.0 %.

The A-form crystals showed endothermic reaction at 175°C in a DSC endothermic test and indicated characteristic peaks at 6.7, 13.4 and 21.5° at 2 *θ* of the X-ray diffraction. Figs. 1, 4 and 7 show the IR (KBr) spectrum, DSC endothermic curve and X-ray diffraction pattern of the crystals, respectively.
IR_{KBr} (cm⁻¹); 3310, 2220, 1634, 1592, 1556, 1417, 1329, 1159, 1118, 1071, 958, 844, 658, 648.

### 5-2

In 50 mL of acetone, 5 g of crude crystals of the compound (I) obtained in Example 2 were dissolved. This solution was refluxed with heating to 57°C with stirring. Then, the resulting mixture was heated to 60°C while adding 50 mL of water dropwise at the same temperature in about an hour. After the dropping was finished, the mixture was further stirred at the same temperature for an hour. Precipitated crystals were collected by filtration and dried at 40°C under vacuum to give 4.12 g of A-form crystals of the compound (I) in a yield of 82.4 %.

The IR (KBr) spectrum, DSC endothermic curve and X-ray diffraction pattern of the crystals were in perfect agreement with those of the A-form crystals of the compound (I) obtained in the above Example 5-1.

### 5-3

In 50 mL of acetone, 5 g of crude crystals of the compound (I) obtained in Example 2 were dissolved. This solution was refluxed with heating to 57°C with stirring. Then, the resulting mixture was heated to 65°C while adding 50 mL of water dropwise at the same temperature in about an hour. After the dropping was finished, the mixture was further stirred at the same temperature for an hour. Precipitated crystals were collected by filtration and dried at 40°C under vacuum to give 3.99 g of A-form crystals of the compound (I) in a yield of 79.8 %.

The IR (KBr) spectrum, DSC endothermic curve and X-ray diffraction pattern of the crystals were in perfect agreement with those of the A-form crystals of the compound (I) obtained in the above Example 5-1.

### 5-4

In 320 mL of cyclohexane, 16 g of crude crystals of the compound (I) obtained in Example 2 were suspended. The suspension was stirred with heating at a temperature between 75°C and a boiling point thereof for 2 hours. Crystals were collected by filtration, washed with 32 ml of cyclohexane and dried under reduced pressure to give 15.2 g of A-form crystals of the compound (I) in a yield of 95.0 %.

The IR (KBr) spectrum, DSC endothermic curve and X-ray diffraction pattern of the crystals were in perfect agreement with those of the A-form crystals of the compound (I) obtained in the above Example 5-1.

### 5-5

In 20 mL of ethyl acetate, 3 g of crude crystals of the compound (I) obtained in Example 2 were dissolved with heating to 40°C. This solution was heated to 60°C with stirring, to which 200 mL of n-heptane was added dropwise at the same temperature in about an hour. After the dropping was finished, the mixture was further stirred at the same temperature for an hour. Precipitated crystals were collected by filtration and dried at 40°C under vacuum to give 1.20 g of A-form crystals of the compound (I) in a yield of 40 %.

The IR (KBr) spectrum, DSC endothermic curve and X-ray diffraction pattern of the crystals were in perfect agreement with those of the A-form crystals of the compound (I) obtained in the above Example 5-1.

### 5-6

In 500 mL of methanol, 5 g of crude crystals of the compound (I) obtained in Example 2 were dissolved. This solution was heated to 60°C with stirring, to which 500 mL of water was added dropwise at the same temperature in about an hour. After the dropping was finished, the mixture was further stirred at the same temperature for an hour. Precipitated crystals were collected by filtration and dried at 40°C under vacuum to give 2.79 g of A-form crystals of the compound (I) in a yield of 55.8 %.

The IR (KBr) spectrum, DSC endothermic curve and X-ray diffraction pattern of the crystals were in perfect agreement with those of the A-form crystals of the compound (I) obtained in the above Example 5-1.

### Example 6

### Preparation of B-form crystals of compound (I)

### 6-1

To 300 mL of isopropyl alcohol, 10.0 g of crude crystals of the compound (I) obtained in Example 2 were added and dissolved by heating to 78°C. This solution was cooled to 25°C with stirring, and then the stirring was continued for 19 hours at the same temperature (22~25 °C). Precipitated crystals were collected by filtration at the same temperature, washed with 120 mL of isopropyl alcohol and then dried under reduced pressure to give 8.87 g of B-form crystals of the compound (I) in a yield of 88.7 %.

The B-form crystals showed endothermic reaction at 92 and 175°C in a DSC endothermic test and indicated characteristic peaks at 6.2, 12.5 and 20.8° at 2 *θ* of the X-ray diffraction. Figs. 2, 5 and 8 show the IR (KBr) spectrum, DSC endothermic curve and X-ray diffraction pattern of the crystals, respectively.
IR _{KBr} (cm⁻¹); 3311, 2217, 1635, 1614, 1594, 1418, 1399, 1322, 1269, 1163, 1125, 1116, 1073, 1020, 970, 843, 666, 659, 592, 525.

### 6-2

In 50 mL of acetone, 5 g of crude crystals of the compound (I) obtained in Example 2 were dissolved. This solution was maintained at 25°C with stirring, to which 50 mL of water was added dropwise at the same temperature in about an hour. After the dropping was finished, the mixture was further stirred at the same temperature for an hour. Precipitated crystals were collected by filtration and dried at 40°C under vacuum to give 4.71 g of B-form crystals of the compound (I) in a yield of 94.2 %.

The IR (KBr) spectrum, DSC endothermic curve and X-ray diffraction pattern of the crystals were in perfect agreement with those of the B-form crystals of the compound (I) obtained in the above Example 6-1.

### 6-3

In 50 mL of acetone, 5 g of crude crystals of the compound (I) obtained in Example 2 were dissolved. This solution was maintained at 30°C with stirring, to which 50 mL of water was added dropwise at the same temperature in about an hour. After the dropping was finished, the mixture was further stirred at the same temperature for an hour. Precipitated crystals were collected by filtration and dried at 40°C under vacuum to give 4.69 g of B-form crystals of the compound (I) in a yield of 93.8 %.

The IR (KBr) spectrum, DSC endothermic curve and X-ray diffraction pattern of the crystals were in perfect agreement with those of the B-form crystals of the compound (I) obtained in the above Example 6-1.

### 6-4

In 50 mL of acetone, 5 g of crude crystals of the compound (I) obtained in Example 2 were dissolved. This solution was maintained at 35°C with stirring, to which 50 mL of water was added dropwise at the same temperature in about an hour. After the dropping was finished, the mixture was further stirred at the same temperature for an hour. Precipitated crystals were collected by filtration and dried at 40°C under vacuum to give 4.64 g of B-form crystals of the compound (I) in a yield of 92.8 %.

The IR (KBr) spectrum, DSC endothermic curve and X-ray diffraction pattern of the crystals were in perfect agreement with those of the B-form crystals of the compound (I) obtained in the above Example 6-1.

### 6-5

To 900 L of acetone, 126 kg of crude wet crystals of the compound (I) obtained in Example 4 were added and dissolved with stirring at room temperature. To this solution, 900 L of water was added dropwise at 30 to 40°C with stirring in an hour. The mixture was stirred at the same temperature for an hour and then cooled to 20 to 30°C. Precipitated crystals were collected by filtration and washed with 120 L of a 50% aqueous acetone solution and then dried at 40°C under vacuum to give 86.1 kg of B-form crystals of the compound (I) in a yield of 75.0 %.

The IR (KBr) spectrum, DSC endothermic curve and X-ray diffraction pattern of the crystals were in perfect agreement with those of the B-form crystals of the compound (I) obtained in the above Example 6. ¹H NMR (CDCl₃, 200MHz) δ: 2.07 (t, J=2.7Hz, 1H), 2.64 (dt, J=2.7 and 7.0Hz, 2H), 2.88 (t, J=7.0Hz, 2H), 7.65 (s,4H), 7.77 (br.s,1H), 15.59 (br.s,1H).
IR (ATR method) (cm⁻¹); 3311, 2217, 1627, 1626, 1589, 1554, 1415, 1321, 1263, 1243, 1160, 1113, 1072, 841, 658.

### 6-6

In 1084 L of acetone, 135.5 kg of crude crystals of the compound (I) obtained in Example 3 were dissolved at room temperature with stirring. The solution was filtered, the residue was washed with 271 L of acetone and the filtrate and the washings were combined. To this solution, 1355 L of water was added dropwise at 30 to 40°C with stirring in about an hour. Then, the mixture was stirred at the same temperature for an hour and cooled to 20 to 30°C. Precipitated crystals were collected by filtration, washed with 270 L of a 50% aqueous acetone solution and then dried at 40°C under vacuum to give 125.4 kg of B-form crystals of the compound (I) in a yield of 92.5%.

The IR (KBr) spectrum, DSC endothermic curve and X-ray diffraction pattern of the crystals were in perfect agreement with those of the B-form crystals of the compound (I) obtained in the above Example 6.

### 6-7

In 30 mL of ethyl acetate, 3 g of crude crystals of the compound (I) obtained in Example 2 were dissolved. This solution was maintained at 30°C with stirring, to which 120 mL of n-heptane was added dropwise at the same temperature in about an hour. After the dropping was finished, the mixture was further stirred at the same temperature for an hour. Precipitated crystals were collected by filtration and dried at 40°C under vacuum to give 1.88 g of B-form crystals of the compound (I) in a yield of 62.7%.

The IR (KBr) spectrum, DSC endothermic curve and X-ray diffraction pattern of the crystals were in perfect agreement with those of the B-form crystals of the compound (I) obtained in the above Example 6-1.

### 6-8

In 500 mL of methanol, 5 g of crude crystals of the compound (I) obtained in Example 2 were dissolved. This solution was maintained at 30°C with stirring, to which 500 mL of water was added dropwise at the same temperature in about an hour. After the dropping was finished, the mixture was further stirred at the same temperature for an hour. Precipitated crystals were collected by filtration and dried at 40°C under vacuum to give 4.23 g of B-form crystals of the compound (I) in a yield of 84.6 %.

The IR (KBr) spectrum, DSC endothermic curve and X-ray diffraction pattern of the crystals were in perfect agreement with those of the B-form crystals of the compound (I) obtained in the above Example 6-1.

### Example 7

### Preparation of C-form crystals of compound (I)

### 7-1

In 100 mL of acetone, 10 g of crude crystals obtained in Example 2 were dissolved with stirring at room temperature. To this solution, 100 mL of water was added dropwise at 14 to 15°C with stirring and the stirring was continued for 2 hours at the same temperature (15°C). Precipitated crystals were collected by filtration at the same temperature, washed with 20 ml of a mixture of acetone and water and then dried under reduced pressure to give 9.35 g of C-form crystals of the compound (I) in a yield of 93.5%.

The C-form crystals showed endothermic reaction at 88 and 174.5 °C in a DSC endothermic test and indicated characteristic peaks at 6.2, 12.4 and 20.2° at 2 *θ* of the X-ray diffraction. Figs. 3, 6 and 9 show the IR (KBr) spectrum, X-ray diffraction pattern and DSC endothermic curve of the crystals, respectively.
IR _{KBr} (cm⁻¹); 3309, 2221, 1590, 1554, 1417, 1388, 1328, 1162, 1118, 1072, 1018, 847, 664, 647.

### 7-2

In 60 mL of acetone, 5 g of crude crystals of the compound (I) obtained in Example 2 were dissolved. This solution was maintained at 5°C with stirring, to which 60 mL of water was added dropwise at the same temperature in about an hour. After the dropping was finished, the mixture was further stirred at the same temperature for an hour. Precipitated crystals were collected by filtration and dried at 40°C under vacuum to give 4.86 g of C-form crystals of the compound (I) in a yield of 97.2 %.

The IR (KBr) spectrum, DSC endothermic curve and X-ray diffraction pattern of the crystals were in perfect agreement with those of the C-form crystals of the compound (I) obtained in the above Example 7-1.

### 7-3

In 50 ml of acetone, 5 g of crude crystals of the compound (I) obtained in Example 2 were dissolved. This solution was maintained at 10°C with stirring, to which 50 mL of water was added dropwise at the same temperature in about an hour. After the dropping was finished, the mixture was further stirred at the same temperature for an hour. Precipitated crystals were collected by filtration and dried at 40°C under vacuum to give 4.84 g of C-form crystals of the compound (I) in a yield of 96.8 %.

The IR (KBr) spectrum, DSC endothermic curve and X-ray diffraction pattern of the crystals were in perfect agreement with those of the C-form crystals of the compound (I) obtained in the above Example 7-1.

### 7-4

In 50 mL of ethyl acetate, 3 g of crude crystals of the compound (I) obtained in Example 2 were dissolved. This solution was maintained at 5°C with stirring, to which 200 mL of n-heptane was added dropwise at the same temperature in about an hour. After the dropping was finished, the mixture was further stirred at the same temperature for an hour. Precipitated crystals were collected by filtration and dried at 40°C under vacuum to give 2.10 g of C-form crystals of the compound (I) in a yield of 70.0 %.

The IR (KBr) spectrum, DSC endothermic curve and X-ray diffraction pattern of the crystals were in perfect agreement with those of the C-form crystals of the compound (I) obtained in the above Example 7-1.

### 7-5

In 1000 mL of methanol, 4 g of crude crystals of the compound (I) obtained in Example 2 were dissolved. This solution was maintained at 5°C with stirring, to which 1000 mL of water was added dropwise at the same temperature in about an hour. After the dropping was finished, the mixture was further stirred at the same temperature for an hour. Precipitated crystals were collected by filtration and dried at 40°C under vacuum to give 3.23 g of C-form crystals of the compound (I) in a yield of 80.8 %.

The IR (KBr) spectrum, DSC endothermic curve and X-ray diffraction pattern of the crystals were in perfect agreement with those of the C-form crystals of the compound (I) obtained in the above Example 7-1.

### Example 8

### Preparation of A-form crystals from B-form crystals of compound (I)

### 8-1

In 600 mL of n-heptane, 50 g of the B-form crystals of the compound (I) obtained in Example 6 were suspended, heated to 88°C and kept stirred at the same temperature for about 5 hours. Then, the mixture was cooled to 45°C with stirring, which was stirred at the same temperature for about 3 hours. Precipitated crystals were collected by filtration at the same temperature, washed with 50 mL of n-heptane and then dried at 40°C under vacuum to give 48.7g of A-form crystals of the compound (I) in a yield of 97.4 %.

The IR (KBr) spectrum, DSC endothermic curve and X-ray diffraction pattern of the crystals were in perfect agreement with those of the A-form crystals of the compound (I) obtained in Example 5.

### 8-2

In 400 mL of a 50% aqueous acetone solution, 20 g of the B-form crystals of the compound (I) obtained in Example 6 were suspended. The suspension was heated to 65°C with stirring, and the stirring was continued for 5 hours at the same temperature. The suspension was filtered at the same temperature to take out crystals, which were dried at 40°C under vacuum to give 15.96 g of A-form crystals of the compound (I) in a yield of 79.8 %.

The IR (KBr) spectrum, DSC endothermic curve and X-ray diffraction pattern of the crystals were in perfect agreement with those of the A-form crystals of the compound (I) obtained in Example 5.

### 8-3

In 600 mL of cyclohexane, 30 g of the B-form crystals of the compound (I) obtained in Example 6 were suspended. The suspension was heated to 72°C with stirring, and the stirring was continued for 5 hours at the same temperature. The suspension was filtered at the same temperature to take out crystals, which were washed with 150 mL of cyclohexane and dried at 40°C under vacuum to give 27.52 g of A-form crystals of the compound (I) in a yield of 91.7 %.

The IR (KBr) spectrum, DSC endothermic curve and X-ray diffraction pattern of the crystals were in perfect agreement with those of the A-form crystals of the compound (I) obtained in Example 5.

### 8-4

In a mixture of 20 mL of ethyl acetate and 200 mL of n-heptane, 3 g of the B-form crystals of the compound (I) obtained in Example 6 were suspended. The suspension was heated to 60°C and stirred at the same temperature for about 5 hours. Then, precipitated crystals were collected by filtration at the same temperature and dried at 40°C under vacuum to give 1.18 g of A-form crystals of the compound (I) in a yield of 39.3 %.

The IR (KBr) spectrum, DSC endothermic curve and X-ray diffraction pattern of the crystals were in perfect agreement with those of the A-form crystals of the compound (I) obtained in Example 5.

### Example 9

### Preparation of A-form crystals from C-form crystals of compound (I)

### 9-1

In 200 mL of a 50% aqueous acetone solution, 10 g of the C-form crystals of the compound (I) obtained in Example 7 were suspended. This suspension was heated to 65°C with stirring, and the stirring was continued at the same temperature for 5 hours. The suspension was filtered at the same temperature to take out crystals, which were dried at 40°C under vacuum to give 7.98 g of A-form crystals of the compound (I) in a yield of 79.8%.

The IR (KBr) spectrum, DSC endothermic curve and X-ray diffraction pattern of the crystals were in perfect agreement with those of the A-form crystals of the compound (I) obtained in Example 5.

### 9-2

In 100 mL of a 50% aqueous methanol solution, 5 g of the C-form crystals of the compound (I) obtained in Example 7 were suspended. This suspension was heated to 60°C with stirring, and the stirring was continued for 15 hours at the same temperature. The suspension was filtered at the same temperature to take out crystals, which were dried at 40°C under vacuum to give 3.91 g of A-form crystals of the compound (I) in a yield of 78.2%.

The IR (KBr) spectrum, DSC endothermic curve and X-ray diffraction pattern of the crystals were in perfect agreement with those of the A-form crystals of the compound (I) obtained in Example 5.

### Example 10

### Preparation of B-form crystals from A-form crystals of compound (I)

### 10-1

In 100 mL of a 50% aqueous acetone solution, 10 g of the A-form crystals of the compound (I) obtained in Example 5 were suspended. The suspension was maintained at 31°C with stirring, and the stirring was continued at the same temperature for 15 hours. The suspension was filtered at the same temperature to take out crystals, which were dried at 40°C under vacuum to give 9.38 g of B-form crystals of the compound (I) in a yield of 93.8%.

The IR (KBr) spectrum, DSC endothermic curve and X-ray diffraction pattern of the crystals were in perfect agreement with those of the B-form crystals of the compound (I) obtained in Example 6.

### 10-2

In 100 mL of isopropyl alcohol, 3 g of the A-form crystals of the compound (I) obtained in Example 5 were suspended. The suspension was maintained at 31°C with stirring, and the stirring was continued for 15 hours at the same temperature. The suspension was filtered at the same temperature to take out crystals, which were dried at 40°C under vacuum to give 2.78 g of B-form crystals of the compound (I) in a yield of 92.7%.

The IR (KBr) spectrum, DSC endothermic curve and X-ray diffraction pattern of the crystals were in perfect agreement with those of the B-form crystals of the compound (I) obtained in Example 6.

### 10-3

In a mixture of 30 mL of ethyl acetate and 120 mL of n-heptane, 3 g of the A-form crystals of the compound (I) obtained in Example 5 were suspended. The suspension was maintained at 30°C with stirring, and the stirring was continued for 15 hours at the same temperature. The suspension was filtered at the same temperature to take out crystals, which were dried at 40°C under vacuum to give 1.80 g of B-form crystals of the compound (I) in a yield of 60.0 %.

The IR (KBr) spectrum, DSC endothermic curve and X-ray diffraction pattern of the crystals were in perfect agreement with those of the B-form crystals of the compound (I) obtained in Example 6.

### Example 11

### Preparation of B-form crystals from C-form crystals of compound (I)

### 11-1

In 100 mL of a 50% aqueous acetone solution, 5 g of the C-form crystals of the compound (I) obtained in Example 7 were suspended. The suspension was heated to 65°C with stirring, and the stirring was continued for 5 hours at the same temperature. Then, the suspension was cooled to 30°C and stirred at the same temperature for about 15 hours. The suspension was filtered to take out crystals, which were dried at 40°C under vacuum to give 4.69 g of B-form crystals of the compound (I) in a yield of 93.8%.

The IR (KBr) spectrum, DSC endothermic curve and X-ray diffraction pattern of the crystals were in perfect agreement with those of the B-form crystals of the compound (I) obtained in Example 6.

### 11-2

In a mixture of 20 mL of ethyl acetate and 200 mL of n-heptane, 3 g of the C-form crystals of the compound (I) obtained in Example 7 were suspended. The suspension was heated to 60°C with stirring, and the stirring was continued for about 5 hours at the same temperature. Then, the suspension was cooled to 30°C and kept stirred at the same temperature for about 5 hours. The suspension was filtered to take out crystals, which were dried at 40°C under vacuum to give 2.18 g of B-form crystals of the compound (I) in a yield of 72.6%.

The IR (KBr) spectrum, DSC endothermic curve and X-ray diffraction pattern of the crystals were in perfect agreement with those of the B-form crystals of the compound (I) obtained in Example 6.

### Industrial Applicability

The present invention is characterized in that chlorocarbonic esters used as an activating reagent in the preparation method of the compound (I) are decomposed into alcohols and carbon dioxide after the reaction, thereby so-called industrial waste is hardly generated. Further, since the preparation method of the compound (I) according to the present invention does not require severe reaction conditions, general-purpose facilities can be used. Accordingly, the preparation method is more suitable for the production of the compound (I) on an industrial scale than a conventional method for preparing the compound (I).

Still further, according to the present invention, A-form crystals, B-form crystals and C-form crystals of the compound (I) may selectively be produced with efficiency by controlling temperature for the recrystallization and/or time for the crystal precipitation during the recrystallization of the compound (I).

## Claims

1. A method for preparing a compound (I) represented by the formula: 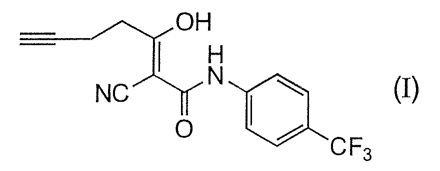 which is **characterized by** reacting a compound (IV) represented by the formula: 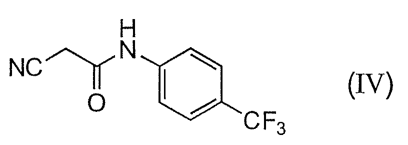 with a mixed acid anhydride of a compound (V) represented by the formula: 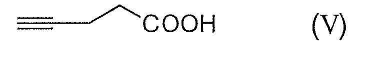 to give the compound (I).

2. A method for preparing a compound (I) represented by the formula: 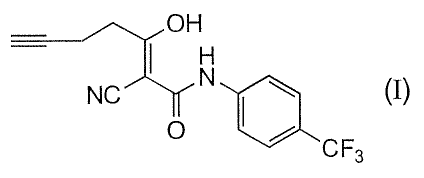 which is **characterized by** reacting a compound (II) represented by the formula: 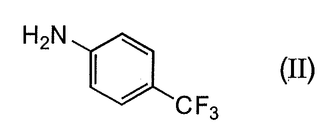 with a carboxylic acid (III) represented by the formula: 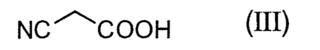 or a reactive derivative at the carboxyl group thereof to give a compound (IV) represented by the formula: 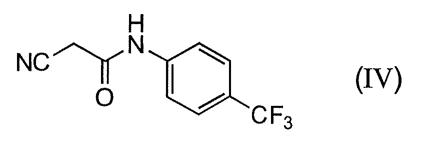 and reacting the resultant compound with a mixed acid anhydride of a compound (V) represented by the formula: 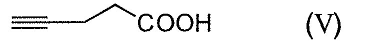 to give the compound (I).

3. The preparation method according to Claim 1 or 2, wherein the mixed acid anhydride of the compound (V) is a mixed acid anhydride with chloro(lower)alkyl carbonate.

4. A method for preparing A-form crystals of the compound (I) described in Claim 1 or 2, which is **characterized by** dissolving the compound (I) in a solvent, maintaining the resultant solution at a temperature from about 55°C to about 95°C while stirring, adding with a poor solvent, if necessary, and then isolating the precipitated crystals.

5. A method for preparing B-form crystals of the compound (I) described in Claim 1 or 2, which is **characterized by** dissolving the compound (I) in a solvent, maintaining the resultant solution at a temperature from about 20°C to about 45°C while stirring, adding with a poor solvent, if necessary, and then isolating the precipitated crystals.

6. A method for preparing C-form crystals of the compound (I) described in Claim 1 or 2, which is **characterized by** dissolving the compound (I) in a solvent, maintaining the resultant solution at a temperature from about 0°C to about 15°C while stirring, adding with a poor solvent, if necessary, and then isolating the precipitated crystals.

7. The method according to any one of Claims 4 to 6, wherein the poor solvent is selected from an aliphatic hydrocarbon such as n-pentane, cyclopentane, n-hexane, cyclohexane, n-heptane or cycloheptane; an aromatic hydrocarbon such as benzene, toluene or xylene; an ethers such as diisopropyl ether; and water.

8. The method according to claim 5, which is **characterized in that** the solution is maintained at a temperature from about 30°C to about 40 °C.

9. The method according to any one of Claims 4 to 6 and 8, wherein the solvent is acetone, and water is added as the poor solvent.

10. The method according to any one of Claims 4 to 6 and 8, wherein the solvent is methanol, and water is added as the poor solvent.

11. The method according to any one of Claims 4 to 6 and 8, wherein the solvent is ethyl acetate, and n-heptane is added as the poor solvent.

12. The method according to Claim 5, wherein the solvent is isopropyl alcohol and no poor solvent is added.

13. A method for converting of B-form crystals or C-form crystals of the compound (I) described in Claims 5 or 6 respectively, or mixture thereof into A-form crystals of the compound (I), which is **characterized by** suspending B-form crystals or C-form crystals of the compound (I) or mixture thereof in a solvent and stirring the resultant suspension at a temperature from about 55°C to about 95°C.

14. A method for converting of A-form crystals of the compound (I) described in Claim 4 into B-form crystals of the compound (I), which is **characterized by** suspending B-form crystals in a solvent and stirring the resultant suspension at a temperature from about 20°C to about 45°C.

15. The method according to Claim 13 or 14, wherein the solvent is an aqueous acetone, aqueous methanol, isopropyl alcohol, cyclohexane, n-heptane or a mixture of ethyl acetate and n-heptane.

16. The method according to Claim 15, wherein the stirring is continued for about 5 hours to about 72 hours.
